# EUROPEAN PATENT SPECIFICATION

(11) **EP 2 939 678 B1**
(45) Date of publication and mention of the grant of the patent: **19.04.2017**
(21) Application number: 13867652.3
(22) Date of filing: 04.11.2013
(51) Int. Cl.: A61K 31/702, A61L 26/00, A61P 17/02, A61F 13/00, A61L 15/10, A61L 15/14, A61K 31/136, A61K 31/185

(54) **THIXOTROPIC FLUID FOR USE AS A CICATRIZING AGENT**
THIXOTROPE FLUID ZUR VERWENDUNG ALS NARBENBILDENDES MITTEL
FLUIDE THIXOTROPE DESTINÉ À LA PREPARATION D'AGENT DE CICATRISATION

(30) Priority: 27.12.2012 AR P012105043
(43) Date of publication of application: 04.11.2015
(73) Proprietor: Vega Villavicencio, Parsival, C.A.B.A. (AR); Battista, Miguel Edgardo, Salta 2575, Provincia de Buenos Aires (AR)
(72) Inventor: Vega Villavicencio, Parsival, C.A.B.A. (AR); Battista, Miguel Edgardo, Salta 2575, Provincia de Buenos Aires (AR)
(74) Representative: Gallego Jiménez, José Fernando
(86) International application number: PCT/PE2013/000010
(87) International publication number: WO 2014/104898

(56) References cited:
- ES-T3- 2 292 711
- US-A- 3 238 100
- US-A- 3 328 259
- US-A- 3 577 516
- US-A- 3 969 498
- US-A1- 2004 018 241
- US-A1- 2007 161 936
- REED B R ET AL: "Cutaneous tissue repair: Practical implications of current knowledge. II", JOURNAL OF THE AMERICAN ACADEMY OF DERMATOLOGY, MOSBY, INC, US, vol. 13, no. 6, 1 December 1985 (1985-12-01), pages 919-941, XP025649979, ISSN: 0190-9622, DOI: 10.1016/S0190-9622(85)70242-3 [retrieved on 1985-12-01]
- MOBACKEN ET AL: "Gentian violet and wound repair", JOURNAL OF THE AMERICAN ACADEMY OF DERMATOLOGY, MOSBY, INC, US, vol. 15, no. 6, 1 December 1986 (1986-12-01), page 1303, XP024101252, ISSN: 0190-9622, DOI: 10.1016/S0190-9622(86)80049-4 [retrieved on 1986-12-01]

## Description

The present invention relates to artificial cicatrization means by means of which a substance is applied to the damaged surfaces, wounds, burns A-AB, after removing the necrotic tissue and disinfecting the damaged or burnt zone.

### 2. PRIOR ART AND PROBLEMS TO BE SOLVED

The technique consists of the removal of the necrotic tissue from the injured zone and subsequent application of the artificial covering mentioned in the title. The preparation causes the formation of an artificial scab on the injured surface.

The problems to be solved are:
1. To replace the use of gauzes since they adhere to the damaged zone and cause lesions in the granulation zone when an attempt is made to remove them, thus causing defective cicatrizations.
2. To avoid potential infections given that protection against external biological agents is provided.
3. To avoid the loss of body fluids and heat, resulting from the loss of the integrity of the mechanical barrier.

The porous artificial scab allows the formation of new tissue, allowing its natural respiration: during its regeneration and bringing about a faster development of cicatrization by avoiding disrupted processes such as the necrosis or the development of anaerobes, without the need to cover and without performing further treatments until the natural detachment of the "Scab."

### 3. AIM OF THE INVENTION

*To protect the lesion against infections and losses of fluids with an artificial scab, and thus the cicatrization is much more rapid and the new tissue is not defective.
*The usual cost of materials decreases, since once the scab is formed, further treatments are not necessary and the expulsion of this scab is natural.

### 4. DETAILED DESCRIPTION OF THE INVENTION

The invention discloses an aqueous thixotropic fluid comprising 1-5 weight% of azosulfamide, 0.004-0.007 weight% of gentian violet, 0.001-0.003 weight% of dexamethasone, 0.5-2 weight% of panthenol, and 0.03-0.06 weight% of gentamycin. The fluid is for topical use as a cicatrizing agent, in particular for the treatment of lesions, wounds or burns. This aqueous thixotropic fluid which forms due to the reaction in the container is produced by the combination of 5 substances; azosulfamide between 1 and 5% + gentian violet between 0.004 and 0.007% + dexamethasone between 0.001 and 0.003% + panthenol between 0.5 and 2% + gentamycin between 0.03 and 0.06% having a red color., The preparation keeps its original properties for two years kept in caramel colored glass closed by screw cap or in pressure plastic at ambient temperature between 12 and 15 degrees centigrade.

In the case of extensive lesions, this red colored liquid is eliminated through the urine producing a reddish coloration without harmful effects, a fact that the users should be alerted to.

The necrotic tissues are removed, and a cleaning, degreasing and drying with petroleum ether is carried out.

The container is first shaken and it is applied on the injured surface with a clean metal spatula or by spray.

It can be applied to the eyes, ear and mucosas without the use of cleaning solvent and without risks.

Once applied to the surface of the living tissue, physicochemical reactions occur by enzymatic catalysis, generating a semicompact web by ionic and covalent bonds between its reactive groups.

This web adheres to the damaged tissues via exposed groups of said tissues.

The diagram below is intended to show the most probable structure.
d-Panthenol oxidizes enzymatically to form pantothenic acid.

This acid can react so as to form part of the coenzyme A or to bring about its bonding to the web mentioned in the scheme.

The sulfonyl groups, the carboxyl groups, the primary or secondary hydroxyl phenol or alcohol groups can react with exposed reactive groups of proteins or amino sugars allowing the attachment of the scab to the damaged skin which presents them at a high concentration.

The forces that confer strength to the structure are intramolecular or intermolecular and of ionic or covalent type.

The bonds of the web which presents numerous crossovers are stabilized between the various layers by hydrophobic interactions, by permanent or induced dipole-dipole hydrogen bonding, and other bonds.

This produces primary, secondary, tertiary and quaternary conformations that confer three dimensionality to the structure.

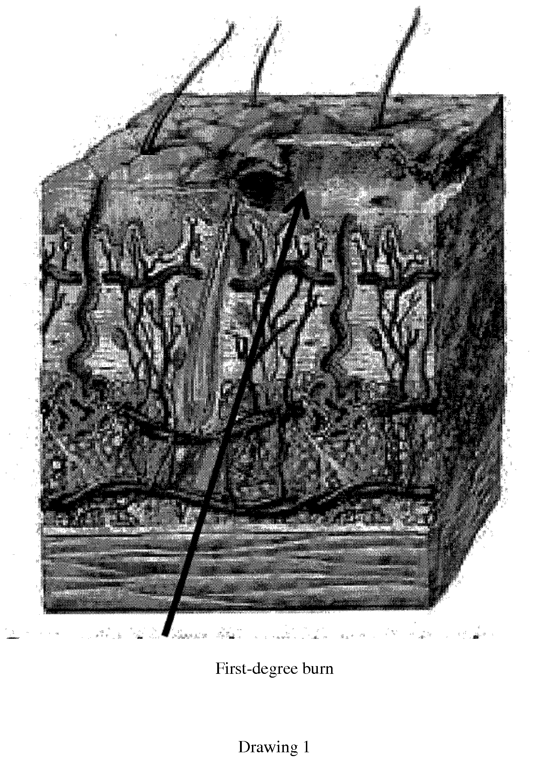

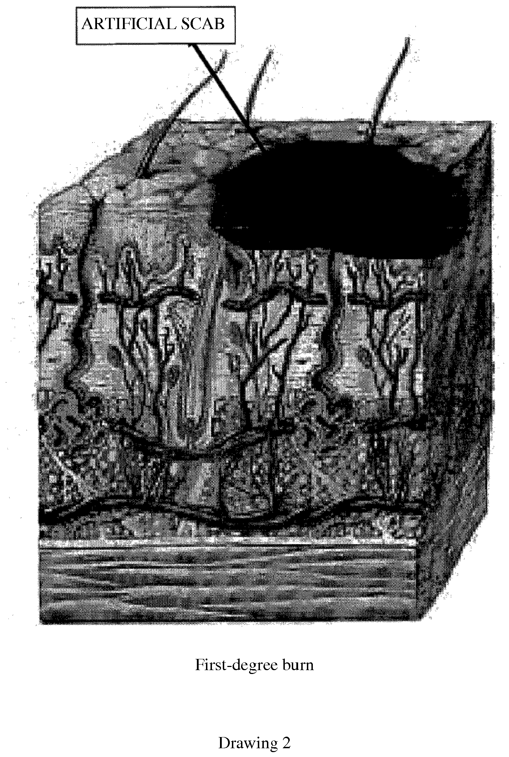

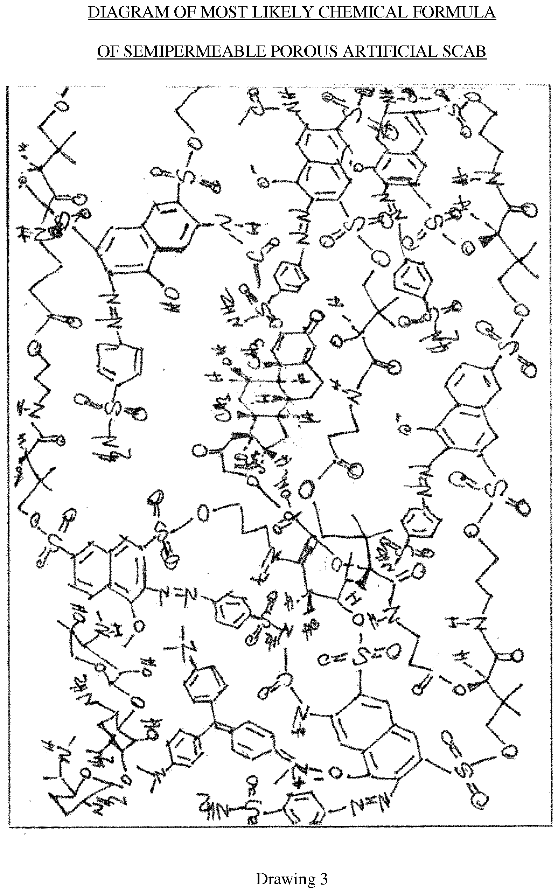

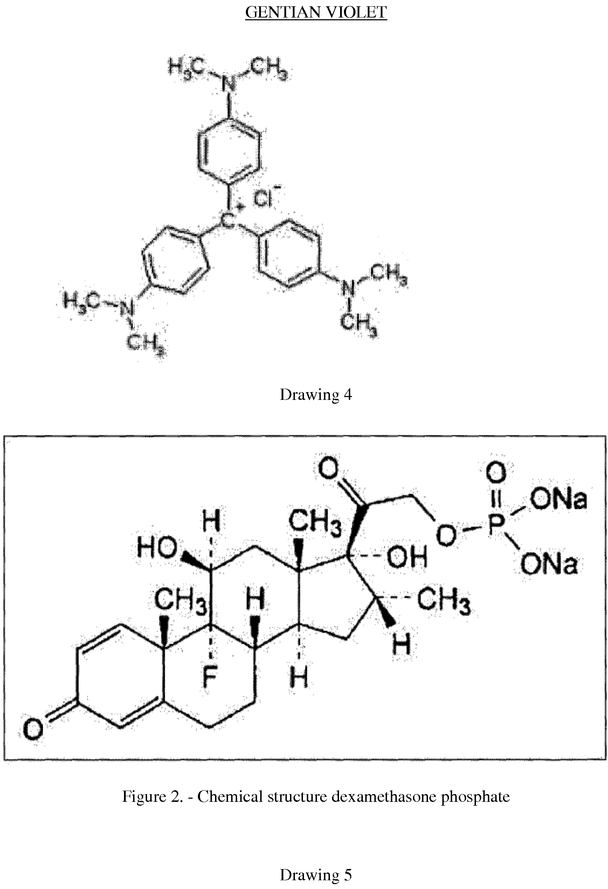

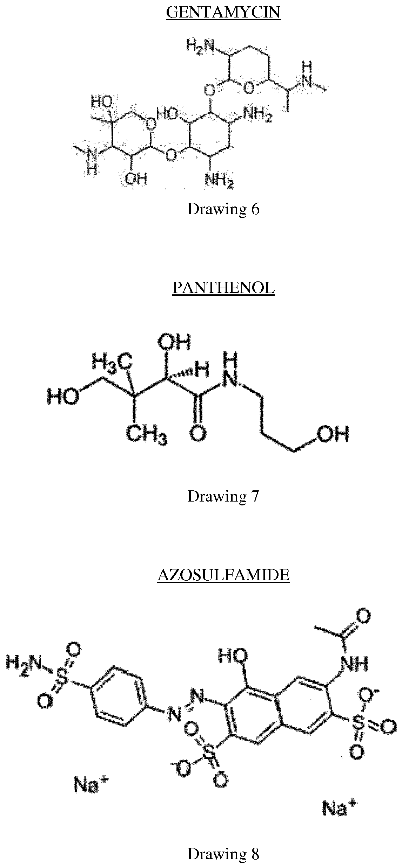

The scope of the invention and the way of producing it in injured tissues (Drawing 1) having been described and specified, the declaration is made to claim as exclusive right and property:
1. - Self-fixing semipermeable porous artificial scab for protecting eroded or injured surfaces, which also allows the growth of new tissues under its protective covering. (SEE DRAWING 2).

It is a composition characterized by the formation of a semipermeable porous artificial scab which is a web produced by the physical reactions between the mentioned compounds at the site of the application, catalyzed by the enzymes present at the injured site.

It would act like a cultured skin but is a chemical polymer in as much as a semipermeable porous artificial scab having these "chemical web" characteristics does not currently exist.

It also prevents the loss of water and electrolytes plasma and proteins.

The five substances described are characterized in that they are applied to a lesion (type A-AB burn or wound) react physicochemically by catalytic action of the damaged tissues thus generating a protective layer which prevents infections and the loss of plasma and proteins.

The new tissues do not lose their original structures due to the protection provided by this layer.

### 5. DESCRIPTION OF THE DRAWINGS

### Drawing 1

Drawing 1 is intended to show a first-degree burn of the skin.

### Drawing 2

This drawing is intended to show the description of the physicochemical reactions that produce a semicompact web by ionic and covalent bonds between its reactive groups; between the different components of the formula one can see peptide bonds, hydrophobic interactions, polar interactions, permanent or induced dipole-dipole bonds, hydrogen bonds, amide, ester, acid base bonds between the various components; this generates primary, secondary, tertiary and quaternary conformations which confer three dimensionality to the structure.

### Drawing 3

Diagram of most likely chemical formula of semipermeable porous artificial scab.

### Drawing 4

Gentian violet: Methyl violet, commonly also called crystal violet or gentian violet, is the name given to a group of chemical compounds used as pH indicators and dyes. Gentian violet of (crystalline violet, methyl violet 10B, hexamethyl pararosaniline chloride) is an antifungal agent.

### Drawing 5

Dexamethasone: Is a fluorinated corticoid having a long duration of action, a high antiinflammatory and immunosuppressant power, and a low mineralocorticoid activity. It inhibits the synthesis of prostaglandins and leukotrienes, mediator substances in the vascular and cellular processes of inflammation and immunological response.

### Drawing 6

Gentamycin: gentamycin is a broad-spectrum aminoglucoside antibiotic with bactericidal action. Mechanism of action: The aminoglucosides are transported actively through the bacterial membrane, they bind irreversibly to one or more specific receptor proteins of the subunit 30S of the bacterial ribosomes and interfere with the initiation complex between mRNA (messenger RNA) and the subunit 30S. The DNA can be read incorrectly, which gives rise to the production of nonfunctional proteins; the polyribosomes separate and are not capable of synthesizing proteins. This gives rise to an accelerated transport of aminoglucosides, as a result of which there is an increase in the rupture of the cytoplasmic membranes of the bacteria and consequent cell death.

### Drawing 7

Panthenol: Panthenol, also referred to as D-pantothenyl alcohol or pantothenol, is a colorless, highly viscous and relatively sticky liquid. It is easily soluble in water and in alcohol (96°). Panthenol dissolves without difficulty in the formulation of hair solutions, provided they are aqueous, hydroalcoholic or hydropropylene-alcoholic solutions.

### Drawing 8

Azosulfamide: The sulfamides are basically derivatives of sulfanilamide which is a structural analog of PABA in which the carboxyl group is replaced by a sulfonyl group (SO2NH2). Thus, the sulfas act as competitive antagonists of PABA, preventing the synthesis of dihydrofolate; in the absence of that metabolite, the synthesis of nucleic acids becomes impossible and as a result bacterial growth is inhibited (bacteriostatic effect).

## Claims

1. Aqueous thixotropic fluid comprising 1-5 weight% of azosulfamide, 0.004-0.007 weight% of gentian violet, 0.001-0.003 weight% of dexamethasone, 0.5-2 weight% of panthenol, and 0.03-0.06 weight% of gentamycin.

2. Fluid according to claim 1 for topical use as cicatrizing agent.

3. Fluid for use according to claim 2 for treating lesions, wounds or burns.

## Patentansprüche

1. Wässriges thixotropes Fluid, umfassend 1-5 Gew.-% Azosulfamid, 0,004-0,007 Gew.-% Gentianaviolett, 0,001-0,003 Gew.-% Dexamethason, 0,5-2 Gew.-% Panthenol und 0,03-0,06 Gew.-% Gentamycin.

2. Fluid nach Anspruch 1 zur topischen Verwendung als narbenbildendes Mittel.

3. Fluid zur Verwendung nach Anspruch 2 zur Behandlung von Läsionen, Wunden oder Verbrennungen.

## Revendications

1. Fluide thixotrope aqueux comprenant 1 à 5 % en poids d'azosulfamide, 0,004 à 0,007 % en poids de violet cristallisé, 0,001 à 0,003 % en poids de dexaméthasone, 0,5 à 2 % en poids de panthénol et 0,03 à 0,06 % de gentamycine.

2. Fluide selon la revendication 1 à usage topique en tant qu'agent de cicatrisation.

3. Fluide destiné selon la revendication 2 au traitement des lésions, des plaies ou des brûlures.
